(19) Europäisches Patentamt
European Patent Office
Office européen des brevets

(11) Veröffentlichungsnummer: **0 284 036 A2**

# (12) EUROPÄISCHE PATENTANMELDUNG

(21) Anmeldenummer: **88104627.0**

(22) Anmeldetag: **23.03.88**

(51) Int. Cl.⁴: **C07C 93/18**, C11D 3/30, D06M 13/46

(30) Priorität: **27.03.87 DE 3710064**

(43) Veröffentlichungstag der Anmeldung: **28.09.88 Patentblatt 88/39**

(84) Benannte Vertragsstaaten: **AT BE CH DE ES FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Hofinger, Manfred, Dr.**
**Rossfeldstrasse 7a**
**D-8269 Burgkirchen(DE)**
Erfinder: **Stühler, Herbert, Dr.**
**Hochfellnstrasse 12**
**D-8269 Burgkirchen(DE)**
Erfinder: **Billenstein, Siegfried, Dr.**
**Samerbergweg 8**
**D-8269 Burgkirchen(DE)**
Erfinder: **Berenbold, Helmut, Dr.**
**Eberleinstrasse 25**
**D-6200 Wiesbaden(DE)**
Erfinder: **Quack, Jochen Meinhard, Dr.**
**Wilhelm-Reuter-Strasse 16**
**D-6239 Eppstein/Taunus(DE)**

## (54) Verfahren zur Herstellung von quaternären Esteraminen und ihre Verwendung.

(57) Es wird ein Verfahren zur Herstellung von quaternären Esteraminen beschrieben, bei dem Glyceride mit Alkanolaminen einer Umesterung unterworfen werden und das erhaltene Umesterungsprodukt quaternisiert wird. Die so erhaltenen Esteramine eignen sich insbesondere als Wäscheweichspüler, Kosmetik-Grundstoff und Weichmacher für Textilien.

EP 0 284 036 A2

# Verfahren zur Herstellung von quaternären Esteraminen und ihre Verwendung

Die Erfindung betrifft ein Verfahren zur Herstellung von quaternären Esteraminen, bei dem von Glyceriden und Alkanolaminen ausgegangen wird. Die Erfindung betrifft ferner die Verwendung der hergestellten quaternären Esteramine.

In der russischen Patentschrift 925 978 (Derwent-Referat Nr. 26938 K/11) ist ein Verfahren zur Herstellung von Esteraminen beschrieben, bei dem Glyceride mit mehrwertigen Alkoholen wie Ethylenglykol, Glycerin, Pentaerythrit und Sorbit, oder mit Aminoalkoholen wie Diethanolamin, Triethanolamin, Trimethylolmelamin und Oxamine umgesetzt werden. Die mit dieser Umesterungsreaktion erhaltenen tensidischen Produkte eignen sich gemäß obengenannter Patentschrift insbesondere als Emulgatoren bei der Bohrung und Aufarbeitung von Erdöl.
Diese bekannten tensidischen Produkte lassen bezüglich ihrer Wirksamkeit noch zu wünschen übrig. Nachteilig ist ferner, daß diese an sich vorteilhaften Esteramine nicht auch auf anderen Gebieten als dem der Erdölgewinnung eine hohe Tensidwirkung zeigen. Gewünscht wären also Esteramine mit vielseitiger Einsetzbarkeit und mit einer jeweils hohen tensidischen Wirkung.

Es wurde nun überraschenderweise gefunden, daß dies dann erreicht wird, wenn Glyceride mit bestimmten Alkanolaminen umgesetzt werden und wenn zusätzlich das erhaltene Umesterungsprodukt quaternisiert wird. Die so erhaltenen quaternären Esteramine stellen sowohl vielseitig einsetzbare als auch jeweils hochwirksame Produkte dar.

Das erfindungsgemäße Verfahren zur Herstellung von quaternären Esteraminen durch Umsetzung von Glyceriden mit Alkanolaminen ist dadurch gekennzeichnet, daß ein Glycerid mit einem Alkanolamin der nachstehenden Formel I

$$(R)_{3-x}\text{-N-}[(CH_2CHR_1O)_n\text{-H}]_x ,$$

in der R eine Alkylgruppe mit 1 bis 4 C-Atomen, x 1, 2 oder 3, n eine ganze Zahl von 1 bis 8, vorzugsweise 1 bis 4, und $R_1$ H oder $CH_3$ ist, wobei die Gruppe $(CH_2CHR_1O)_n$ auch Ethylenoxid-und Propylenoxid-Einheiten enthalten kann, umgesetzt wird und das erhaltene Umsetzungsprodukt quaternisiert wird.

Als Alkanolamine werden vorzugsweise solche eingesetzt, die sich aus Formel I ergeben, wenn die Gruppe $(CH_2CHR_1O)_n$aus nur Ethylenoxid-Einheiten oder aus nur Propylenoxid-Einheiten besteht und dabei n eine ganze Zahl von 1 bis 4 ist, oder wenn sie aus einem 1 bis 4 Ethylenoxid-Einheiten enthaltenden Ethylenoxid-Block und einem 1 bis 4 Propylenoxid-Einheiten enthaltenden Propylenoxid-Block besteht. Bezüglich R in Formel I sei gesagt, daß im Falle von mehreren Alkylgruppen diese gleich oder verschieden sein können. Die folgenden Alkanolamine werden besonders bevorzugt eingesetzt:
Monomethyl-oder Monopropylamin, ethoxyliert mit 2 bis 4 mol Ethylenoxid (pro mol Amin), Dimethyl-oder Dipropylamin, ethoxyliert mit 1 bis 2 mol Ethylenoxid, Triethanol-oder Tripropanolamin und Triethanol-oder Tripropanolamin, ethoxyliert mit 1 bis 3 mol Ethylenoxid.

Die erfindungsgemäß einzusetzenden Alkanolamine sind wohlbekannte Verbindungen. Im Falle von Ethoxylaten und/oder Propoxylaten wird es sich in der Regel um Gemische homologer Alkanolamine handeln Es versteht sich auch von selbst, daß im Falle von Di-und Trialkanolaminen die 2 oder die 3 Ethoxylat-und/oder Propoxylat-Reste in der Regel nicht gleich (lang) sind.

Die erfindungsgemäß einzusetzenden Glyceride, das sind Fettsäureglycerinester, entsprechen der nachstehenden Formel II

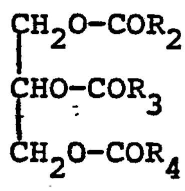

,

in der $R_2$, $R_3$ und $R_4$ gleich oder verschieden, geradkettig oder verzweigt, gesättigt oder ungesättigt sein können. Bevorzugt handelt es sich um geradkettige Alkyl-oder Alkenylgruppen mit 5 bis 23 C-Atomen, vorzugsweise 11 bis 17 C-Atomen, wobei die Alkenylgruppen vorzugsweise 1 bis 3 Doppelbindungen aufweisen. Die Acylgruppen $R_2CO$, $R_3CO$ und $R_4CO$ leiten sich also vorzugsweise von den folgenden Carbonsäuren (Fettsäuren) ab:
Alkansäuren mit 6 bis 24, vorzugsweise 12 bis 18 C-Atomen, wie Capronsäure, Caprylsäure, Pelargonsäure, Caprinsäure, Undecansäure, Laurinsäure, Myristinsäure, Pentadecansäure, Palmitinsäure, Stearinsäure, Arachinsäure (Eicosansäure) und Behensäure;
Alkensäuren, Alkadiensäuren und Alkatriensäuren wie Lauroleinsäure, Myristoleinsäure, Palmitoleinsäure, Ölsäure, Erucasäure, Linolsäure, Eicosadiensäure und Linolensäure. Bevorzugte Glyceride für das erfindungsgemäße Verfahren sind vor allem die natürlichen (pflanzlichen) oder tierischen Fette und Öle, die Gemische aus überwiegend Triglyceriden darstellen. Als Beispiele für pflanzliche Fette und Öle seien genannt: Olivenöl, Cocosfett, Palmkernfett, Palmöl, Erdnußöl, Rüböl, Ricinusöl, Sesamöl, Sonnenblumenöl, Sojaöl, Hanföl, Kakaobutter und Pflanzentalge; als Beispiele für tierische Fette und Öle seien genannt: Rindertalg, Schweinefett, Knochenfett, Hammeltalg, Japantalg, Walöl und andere Fischöle. Ebenso eingesetzt werden können einheitliche Triglyceride oder Mischungen davon, sei es, daß diese aus natürlichen Fetten isoliert oder auf synthetischem Wege gewonnen wurden. Hier seien beispielsweise genannt: Trilaurin, Trimyristin, Tripalmitin, Tristearin, Triolein, Trilinolein und Trilinolenin oder gemischte Glyceride, beispielsweise Palmitodistearin, Distearoolein, Dipalmitoolein und Myristopalmitostearin, Von den genannten Glyceriden sind die im Handel erhältlichen technischen Produkte wie raffinierter Talg, gehärteter Talg oder Cocosfett besonders bevorzugt.

Die erfindungsgemäße Umsetzung (Umesterungsreaktion) von einem Alkanolamin mit einem Glycerid wird bei einer Temperatur von 120 bis 200 °C, vorzugsweise 130 bis 170 °C durchgeführt. Es ist möglich, die Umsetzung in Gegenwart von sauren Katalysatoren durchzuführen. Geeignete saure Katalysatoren sind: Halogenwasserstoffsäuren wie Salzsäure; Phosphorsäuren wie unterphosphorige Säure oder Orthophosphorsäure; Schwefelsäure und Sulfonsäuren wieMethansulfonsäure, para-Toluolsulfonsäure oder Dodecylbenzolsulfonsäure. Bevorzugt sind Phosphorsäuren und Sulfonsäuren. Neben diesen sauren Verbindungen sind auch monomere oder polymere Titan- oder Zirkonverbindungen als Katalysator geeignet, wobei die Titanverbindungen aus Zweckmäßigkeitsgründen bevorzugt sind. Als Titanverbindungen sind jene aus der Gruppe der Alkyltitanate (monomere Alkyltitanate oder Titansäureester) und Alkylpolytitanate (polymere Alkyltitanate oder polymere Titansäureester) bevorzugt. Alkyltitanate der Formel $Ti(OR)_4$, worin R ein Alkylrest mit 1 bis 18 Kohlenstoffatomen ist, vorzugsweise mit 1 bis 6 Kohlenstoffatomen, und Alkylpolytitanate der Formel $RO-[Ti(OR)_2O]_n-R$, worin R die obengenannte Bedeutung hat und n eine (ganze) Zahl von 1 bis 10 ist, vorzugsweise von 4 bis 7, sind besonders bevorzugte Titan-Katalysatoren; sowohl in der ersten als auch in der zweiten Formel sind die Alkylreste vorzugsweise gleich. Die Menge an saurem Katalysator oder an Titan-oder Zirkonverbindung als Katalysator beträgt im allgemeinen 0,05 bis 1 Gew.-%, vorzugsweise 0,1 bis 0,5 Gew.-%, bezogen auf das Gewicht des eingesetzten Glycerids. Je nach Reaktionstemperatur und Art der Reaktionskomponenten wird die Umsetzung drucklos oder unter dem (insbesondere aufgrund des eingesetzten Alkanolamins) sich einstellenden Druck ablaufen. Es ist bevorzugt, die Umsetzung ohne Verwendung eines Lösungsmittels, das heißt in Substanz, durchzuführen. Das Molverhältnis der beiden Reaktionskomponenten Alkanolamin und Glycerid richtet sich nach dem angestrebetn Umesterungsprodukt, ob also das Umesterungsprodukt überwiegend Monoester, Diester oder Triester enthalten soll. Erfindungsgemäß ist es bevorzugt, die Umesterungsreaktion auf Monoester-und/oder Diester-Bildung, vorzugsweise auf Diester-Bildung, einzustellen. Wird ein Umesterungsprodukt aus überwiegend Monoester angestrebt, so ist ein Molverhältnis von etwa 1 mol Glycerid zu etwa 3 mol Alkanolamin vorteilhaft; soll das Umesterungsprodukt aus überwiegend Diester bestehen, ist ein Molverhältnis von etwa 1 mol Glycerid zu etwa 1,5 mol Alkanolamin vorteilhaft.

Die Umesterungsreaktion wird zweckmäßigerweise durch gaschromatographische Analyse verfolgt. Nachdem es sich um eine Gleichgewichtsreaktion handelt, wird man die Reaktion nach Erreichung des Maximums an dem angestrebten Ester abbrechen (zum Beispiel durch Abkühlen des Inhaltes des Reaktionsgefäßes). Die Reaktionszeit liegt im allgemeinen im Bereich von 5 bis 20 Stunden. Das erhaltene Umsetzungsprodukt, das gegebenenfalls mit Wasser gewaschen wird, enthält neben den gebildeten Esterverbindungen in der Regel auch noch mehr oder weniger große Anteile an nichtumgesetzten Ausgangsverbindungen und an gebildetem Glycerin. Es stellt ein bei Raumtemperatur festes, farblos bis leicht gelb gefärbtes Produkt dar.

Gemäß Erfindung wird das erhaltene Umesterungsprodukt einer Quaternisierungsreaktion unterworfen. Die Quaternisierung kann durch Reaktion des Umesterungsproduktes a) mit alkylierenden Quaternisierungsmitteln wie Methylchlorid, Dimethylsulfat, Diethylsulfat und Benzylchlorid, oder b) mit Ethylenoxid, Propylenoxid oder einer Mischung davon, vorzugsweise mit Ethylenoxid, in Gegenwart einer zur Salzbildung äquimolaren Menge (entsprechend der mole der Stickstoffatome) Carbonsäure oder Mineralsäure durchgeführt werden. Die beiden Quaternisierungsarten werden bei einer Temperatur von 60 bis 100 °C, vorzugsweise 75 bis 85 °C, durchgeführt, wobei es vorteilhaft ist, einen Reaktionsdruck von bis zu 6 bar nicht zu überschreiten. Sie werden in Substanz, vorzugsweise aber unter Verwendung einesLösungsmittels durchgeführt; geeignete Lösungsmittel sind niedrige Alkanole mit einem Siedepunkt, der oberhalb der Quaternisierungstemperatur liegt. Geeignete Mineralsäuren zur Quaternisierung mit Ethylenoxid und/oder Propylenoxid, vorzugsweise Ethylenoxid, sind Phosphorsäuren, vorzugsweise die Phosphorsäure ($H_3PO_4$). Geeignete Carbonsäuren sind

aliphatische Carbonsäuren mit 1 bis 6 C-Atomen wie Ameisensäure, Essigsäure, Propionsäure und Buttersäure, aliphatische Hydroxycarbonsäuren mit 1 bis 6 C-Atomen und mit 1 bis 3 Hydroxylgruppen wie Glycolsäure und Milchsäure, und aromatische Carbonsäuren wie Benzoesäure und Salicylsäure. Die Carbonsäure kann auch eine, gegebenenfalls OH-substituierte, Di-oder Tricarbonsäure sein wie Bernsteinsäure, Malon-, Malein-, Fumar-, Äpfel-, Wein-und Zitronensäure.

Bevorzugte Säuren sind Essigsäure, Propionsäure, Milchsäure und Phosphorsäure. Die genannten Alkylenoxide werden zweckmäßigerweise in einer Menge von 3 bis 5 mol pro mol Stickstoff im zu quaternisierenden Produkt eingesetzt. Die unter a) genannten Quaternisierungsmittel werden in einer Menge von 1 bis 1,5 mol pro mol Stickstoff eingesetzt. Die Reaktionszeit liegt bei beiden Quaternisierungsarten im Bereich von 5 bis 25 Stunden. Der Quaternisierungsgrad des erhaltenen quaternären Reaktionsproduktes kann durch Zweiphasentitration mit Natriumdodecylsulfat bei pH 1 bis 2, und pH 10 ermittelt werden.

Die nach dem erfindungsgemäßen Verfahren erhaltenen quaternären Esteramine stellen farblose bis leicht gelb gefärbte, bei Raumtemperatur pastöse bis fest vorliegende Produkte dar. Ihr Quaternisierungsgrad liegt im Bereich von vorzugsweise 70 bis 100 %. Sie weisen hohe tensidische Wirkung auf und sind vielseitig einsetzbar. Sie stellen insbesondere überraschendwirksame Wäscheweichspüler, Kosmetik-Grundstoffe, vorzugsweise für die Bereitung von Haar-Nachwaschmitteln, und Weichmacher für Textilien aus künstlichen und/oder natürlichen Fasern dar. Sie zeichnen sich ferner durch eine unerwartet gute Emulgierbarkeit aus, wodurch unter anderem ihre Einarbeitbarkeit bei der Bereitung von Formulierungen wesentlich erleichtert und beschleunigt wird. Für den Einsatz als Wäscheweichspüler und Weichmacher haben sich jene Quaternisierungsprodukte als besonders geeignet erwiesen, die im wesentlichen aus Diestern bestehen, während die im wesentlichen Monoester enthaltenden Produkte für den Einsatz in der Kosmetik besonders geeignet sind.

Die Erfindung wird nun an Beispielen noch näher erläutert.

Beispiel 1

a) Herstellung eines Umesterungsproduktes aus Glycerintristearat und N-Methyldiethanolamin:
In einem 1-l-Rührkolben, versehen mit Kühler, Gaseinleitung und Heizung, werden 220,3 g (0,25 mol) Glycerintristearat und 44,0 g (0,375 mol) Methyldiethanolamin vorgelegt. Die Mischung wird unter Schutzgasatmosphäre (Stickstoff) auf 160 °C erhitzt und bei dieser Temperatur, bei der die Kondensationsreaktion (Umesterungsreaktion) abläuft, unter Rühren gehalten. Nach 8,5stündiger Reaktionszeit wird das Produktgemisch per Gaschromatographie analysiert, wobei ein Anteil von 40,7 Gew.-% an N-Methyldiethanolaminmono-und -distearat festgestellt wird.

b) Quaternisierung des unter a) erhaltenen Kondensationsproduktes (Umesterungsproduktes) mit Methylchlorid:
181,2 g des Kondensationsproduktes (0,25 mol, bezogen auf die ermittelte Aminzahl) werden mit 34,2 g Isopropanol (Lösungsmittel) gemischt. Die Mischung wird nach Schutzgas-Spülung auf 75 °C erhitzt und unter Rühren mit 12,6 g (0,25 mol) Methylchlorid versetzt. Die Quaternisierungs-Reaktion ist bei der Temperatur von etwa 75 °C und einem maximalen Druck von 6 bar nach 7 Stunden beendet. Der Quaternisierungsgrad des Produktes (der quaternären Esteramine), der aus der analytischen Bestimmung von restlichem freiem Amin und Aminsalz errechnet wird, beträgt 98 %.

Beispiel 2

a) Herstellung eines Umesterungsproduktes aus Glycerintristearat und N-Butyldiethanolamin:
Im Rührkolben von Beispiel 1 werden 528,7 g (0,6 mol) Glycerintristearat und 148,8 g (0,9 mol)-Butyldiethanolamin vorgelegt. Die Mischung wird unter Schutzgasatmosphäre auf 160 °C erhitzt und bei dieser Temperatur, bei der die Kondensationsreaktion abläuft, unter Rühren gehalten. Nach 15stündiger Reaktionszeit wird das Produktgemisch per Gaschromatographie analysiert, wobei ein Anteil von 37,6 Gew.-% an N-Butyldiethanolaminmono-und -distearat festgestellt wird.

b) Quaternisierung des unter a) erhaltenen Kondensationsproduktes mit Methylchlorid:
200,0 g des Kondensationsproduktes (0,25 mol, bezogen auf die ermittelte Aminzahl) werden mit 37,5 g Isopropanol gemischt. Die Mischung wird nach Schutzgasspülung auf 75 °C erhitzt und unter Rühren mit 12,6 g (0,25 mol) Methylchlorid versetzt. Die Quaternisierungsreaktion ist bei der Temperatur von 75 bis 80 °C und einem maximalen Druck von 6 bar nach 7 Stunden beendet. Der Quaternisierungsgrad des Produktes, ermittelt wie in Beispiel 1, beträgt 80 %.

Beispiel 3

a) Herstellung eines Umesterungsproduktes aus Glycerintristearat und Triethanolamin:
Im Rührkolben von Beispiel 1 werden 282,0 g (0,32 mol) Glycerintristearat und 76,1 g (0,51 mol) Triethanolamin vorgelegt. Die Mischung wird unter Schutzgasatmosphäre auf 160 °C erhitzt und bei dieser Temperatur, bei der die Kondensationsreaktion abläuft, unter Rühren gehalten. Nach 15stündiger Reaktionszeit wird das Produktgemisch per Gaschromatographie analysiert, wobei ein Anteil von 52,2 Gew.-% Triethanolaminmono-und Triethanolamindistearat mit wenig Triethanolamintristearat festgestellt wird.

b) Quaternisierung des unter a) erhaltenen Kondensationsproduktes mit Methylchlorid:
178,6 g des Kondensationsproduktes (0,35 mol, bezogen auf die ermittelte Aminzahl) werden mit 34,3 g Isopropanol gemischt. Die Mischung wird nach Schutzgasspülung auf 75 °C erhitzt und unter Rühren mit 17,7 g (0,35 mol) Methylchlorid versetzt. Die Quaternisierungsreaktion ist bei der Temperatur von 75 bis 80 °C und einem maximalen Druck von 6 bar nach 20 Stunden beendet.
Der Quaternisierungsgrad des Produktes, ermittelt wie in Beispiel 1, beträgt 95 %.

Beispiel 4

a) Herstellung eines Umesterungsproduktes aus Glycerintristearat und N-Dimethylethanolamin:
Im Rührkolben von Beispiel 1 werden 308,4 g (0,35 mol) Glycerintristearat und 93,6 g (1,05 mol) Dimethylethanolamin vorgelegt. Die Mischung wird unter Schutzgasatmosphäre auf 130 °C erhitzt und bei dieser Temperatur, bei der die Kondensationsreaktion abläuft, unter Rühren gehalten. nach 20stündiger Reaktionszeit wird das Produktgemisch per Gaschromatographie analysiert, wobei ein Anteil von 35,7 Gew.-% N-Dimethylethanolaminmonostearat festgestellt wird.

b) Quaternisierung des unter a) erhaltenen Kondensationsproduktes mit Methylchlorid:
274,0 g des Kondensationsproduktes (0,6 mol, bezogen auf die ermittelte Aminzahl) werden mit 53,7 g Isopropanol gemischt. Die Mischung wird nach Schutzgasspülung auf 80 °C erhitzt und unter Rühren mit 30,3 g (0,6 mol) Methylchlorid versetzt. Die Quaternisierungsreaktion ist bei der Temperatur von etwa 80 °C und einem maximalen Druck von 6 bar nach 12 Stunden beendet.
Der Quaternisierungsgrad des Produktes, ermittelt wie in Beispiel 1, beträgt 99 %.

Beispiel 5

a) Herstellung eines Umesterungsproduktes aus gehärtetem Cocosfett und N-Dimethylethanolamin:
Im Rührkolben von Beispiel 1 werden 110,1 g (0,17 mol) Cocosfett und 45,5 g (0,51 mol) Dimethylethanolamin vorgelegt. Die Mischung wird unter Schutzgasatmosphäre auf 135 °C erhitzt und bei dieser Temperatur, bei der die Kondensationsreaktion abläuft, unter Rühren gehalten. Nach 5stündiger Reaktionszeit wird das Produktgemisch per Gaschromatographie analysiert, wobei ein Anteil von 44,6 Gew.-% N-Dimethylethanolaminmonococoat festgestellt wird.

b) Quaternisierung des unter a) erhaltenen Kondensationsproduktes mit Methylchlorid:
120,0 g des Kondensationsproduktes (0,38 mol, bezogen auf die ermittelte Aminzahl) werden mit 25,6 g Isopropanol gemischt. Die Mischung wird nach Schutzgasspülung auf 80 °C erhitzt und unter Rühren mit 19,3 g (0,38 mol) Methylchlorid versetzt. Die Quaternisierungsreaktion ist bei der Temperatur von etwa 80 °C und einem maximalen Druck von 6 bar nach 6 h beendet.
Der Quaternisierungsgrad des Produktes, ermittelt wie in Beispiel 1, beträgt 99 %.

## Ansprüche

1. Verfahren zur Herstellung von quaternären Esteraminen durch Umsetzung von Glyceriden mit Alkanolaminen, dadurch gekennzeichnet, daß ein Glycerid mit einem Alkanolamin der nachstehenden Formel I

$$(R)_{3-x}\text{-N-}[(CH_2CHR_1O)_n\text{-H}]_x ,$$

in der R eine Alkylgruppe mit 1 bis 4 C-Atomen, x 1, 2 oder 3, n eine ganze Zahl von 1 bis 8, und $R_1$ H oder $CH_3$ ist, wobei die Gruppe $(CH_2CHR_1O)_n$ auch Ethylenoxid-und Propylenoxid-Einheiten enthalten kann, umgesetzt wird und das erhaltene Reaktionsprodukt quaternisiert wird.

2. Verfahren nach Anspruch 1, dadurch gekennzeichnet, daß als Alkanolamine solche eingesetzt werden, die sich aus Formel I ergeben, wenn die Gruppe $(CH_2CHR_1O)_n$ aus nur Ethylenoxid-Einheiten oder aus nur Propylenoxid-Einheiten besteht und n eine ganze Zahl von 1 bis 4 ist, oder wenn sie aus einem 1 bis 4 Ethylenoxid-Einheiten enthaltenden Ethylenoxid-Block und einem 1 bis 4 Propylenoxid-Einheiten enthaltenden Propylenoxid-Block besteht.

3. Verfahren nach Anspruch 1, dadurch gekennzeichnet, daß als Alkanolamine Monomethyl- oder Monopropylamin, ethoxyliert mit 2 bis 4 mol

Ethylenoxid, Dimethyl-oder Dipropylamin, ethoxyliert mit 1 bis 2 mol Ethylenoxid, Triethanol-oder Tripropanolamin oder Triethanol-oder Tripropanolamin, ethoxyliert mit 1 bis 3 mol Ethylenoxid, eingesetzt werden.

4. Verfahren nach einem oder mehreren der Ansprüche 1 bis 3, dadurch gekennzeichnet, daß als Glyceride solche Fettsäureglycerinester eingesetzt werden, deren Acylgruppen die Capronsäure, Caprylsäure, Pelargonsäure, Caprinsäure, Undecansäure, Laurinsäure, Myristinsäure, Pentadecansäure, Palmitinsäure, Stearinsäure, Arachinsäure, Behensäure, Lauroleinsäure, Myristoleinsäure, Palmitoleinsäure, Ölsäure, Erucasäure, Linolsäure, Eicosadiensäure oder die Linolensäure enthalten.

5. Verfahren nach einem oder mehreren der Ansprüche 1 bis 3, dadurch gekennzeichnet, daß als Glyceride pflanzliche oder tierische Fette und Öle aus im wesentlichen Triglyceriden eingesetzt werden.

6. Verfahren nach einem oder mehreren der Ansprüche 1 bis 3, dadurch gekennzeichnet, daß als Glyceride technische Produkte, bestehend im wesentlichen aus raffiniertem Talg, gehärtetem Talg oder Cocosfett eingesetzt werden.

7. Verfahren nach einem oder mehreren der Ansprüche 1 bis 6, dadurch gekennzeichnet, daß die Umsetzung von Alkanolamin und Glycerid bei einer Temperatur von 120 bis 200 °C durchgeführt wird.

8. Verfahren nach einem oder mehreren der Ansprüche 1 bis 7, dadurch gekennzeichnet, daß das Alkanolamin und das Glycerid zur Erreichung eines Umesterungsproduktes aus im wesentlichen Monoester im Molverhältnis von 3 : 1 und zur Erreichung eines Umesterungsprodukts aus im wesentlichen Diester im Molverhältnis von 1,5 : 1 eingesetzt werden.

9. Verfahren nach einem oder mehreren der Ansprüche 1 bis 8, dadurch gekennzeichnet, daß das Umesterungsprodukt aus Alkanolamin und Glycerid mit einem alkylierenden Quaternisierungsmittel oder mit Ethylenoxid, Propylenoxid oder Gemischen dieser beiden Alkylenoxide und einer zur Salzbildung äquimolaren Menge von einer Carbonsäure oder Mineralsäure bei einer Temperatur von 60 bis 100 °C auf einen Quaternisierungsrad von 70 bis 100 % quaternisiert wird.

10. Verwendung der nach den Ansprüchen 1 bis 9 hergestellten quaternären Esteramine als Wäscheweichspüler, Kosmetik-Grundstoff oder Weichmacher für Textilien